# FASCICULE DE BREVET EUROPEEN

(11) **EP 2 490 607 B1**
(45) Date de publication et mention de la délivrance du brevet: **04.05.2016**
(21) Numéro de dépôt: 10787498.4
(22) Date de dépôt: 19.10.2010
(51) Int. Cl.: A61B 17/70

(54) **DISPOSITIF DE STABILISATION DYNAMIQUE INTERLAMAIRE**
VORRICHTUNG ZUR DYNAMISCHEN INTERLAMINAREN STABILISIERUNG
DEVICE FOR DYNAMIC INTERLAMINAR STABILIZATION

(30) Priorité: 23.10.2009 FR 0905096
(43) Date de publication de la demande: 29.08.2012
(73) Titulaire: Biospine Implants, 33600 Pessac (FR)
(72) Inventeur: FORTIN, Frédéric, F-33600 Pessac (FR); ROBIN, Johann, F-33170 Bègles (FR); SENNEQUIER, Brice, F-33600 Pessac (FR)
(86) Numéro de dépôt international: PCT/FR2010/000694
(87) Numéro de publication internationale: WO 2011/048287

(56) Documents cités:
- WO-A2-2007/087535
- FR-A1- 2 884 136
- JP-A- 10 277 070
- US-A1- 2006 271 044

## Description

### Domaine de l'invention

La présente invention concerne un dispositif de stabilisation dynamique interlamaire, se plaçant entre deux vertèbres du rachis lombaire. Elle permet de stabiliser l'articulation intervertébrale, apportant un amortissement tout en conservant la mobilité et en respectant la courbure de la lordose anatomique normale; et elle permet même d'effectuer des corrections de scoliose.

### ART ANTÉRIEUR

Le brevet US20069204150 décrit et revendique un dispositif de stabilisation dynamique interlamaire, dont la particularité principale est d'être réglable suivant un axe de translation vertical. Il s'accroche aux vertèbres et joue aussi le rôle d'un stabilisateur dynamique rigide ou semi-rigide. Il ne possède pas les capacités d'amortissement qu'offre la présente invention. Contrairement à celui de l'antériorité, notre dispositif de stabilisation dynamique interlamaire est souple grâce à la présence d'un moyen essentiel viscoélastique. Le dispositif de l'antériorité présente des inconvénients, car il ne permet pas de réaliser certains mouvements vertébraux en raison de blocages imposés par ce dispositif comportant des tiges bloquées par des vis. La restauration complète de tous les mouvements anatomiques n'est pas obtenue, notamment pour tous les mouvements polyaxiaux.

De plus, le dispositif de l'antériorité comporte des pièces réglables bloquées par des vis qui peuvent se desserrer ce qui peut être préjudiciable pour le patient, ceci pouvant entraîner des complications nécessitant des interventions supplémentaires.

Au contraire, le dispositif de stabilisation dynamique de la présente invention est monobloc, il est assemblé par exemple par soudure sans aucune vis, et il comporte un moyen essentiel viscoélastique qui va jouer le rôle d'un amortisseur autorisant tous les mouvements polyaxiaux liés aux sollicitations dynamiques appliquées sur l'articulation intervertébrale.

Les dysfonctionnements et les pertes de performance dynamique sont improbables contrairement au dispositif de l'art antérieur, car notre dispositif ne peut pas se désolidariser.

Pour éviter les risques précédemment décrits existant dans l'antériorité notamment de desserrage, le dispositif de la présente invention présente une gamme de produits, dont les dimensions géométriques sont adaptables à toutes les anatomies.

Le brevet WO 2007/087535 A2 décrit un système de stabilisation dynamique comportant un amortisseur viscoélastique dans lequel pénètrent à chaque extrémité un piston relié à une fixation vertébrale. Dans la forme de réalisation préférentielle, l'élément viscoélastique est percé à chaque extrémité pour guider les 2 pistons dans le même axe et amortir les sollicitations mécaniques appliquées aux fixations vertébrales transmises par chaque piston. Il n'y a aucun jeu fonctionnel. Il est clair que les perçages à chaque extrémité de l'amortisseur guidant des tiges soumises à des efforts de directions différentes sont autant d'amorces de rupture potentiels qu'il est indispensable d'éviter dans la conception d'un dispositif durable de stabilisation dynamique. Dans une autre forme de réalisation, l'amortisseur 'adhère' aux fixations vertébrales et dans ce cas, on peut supposer que la liaison avec les éléments de fixation aux vertèbres n'est pas assurée.
Notre invention répond à ces problèmes non résolus en évitant aux moyens de notre dispositif de se disloquer entre eux, en assurant la liaison du dispositif de stabilisation dynamique avec les lames vertébrales et en faisant travailler l'élément viscoélastique dans des conditions optimales indispensable à sa longévité.

Le brevet JP 10 277070 A décrit un système dynamique fixé par des vis sur les vertèbres. Ce système comporte un piston pénétrant à l'intérieur d'un cylindre. Le piston peut coulisser librement et prendre un angle avec la direction du déplacement. Il n'y a pas d'amortisseur viscoélastique mais un ressort jouant le rôle d'amortisseur. Les éléments métalliques composants cette invention et plus particulièrement la tête du piston en contact avec le corps cylindrique vont frotter sur la surface interne de ce dernier pendant les mouvements du rachis, créant des débris métalliques préjudiciables à la longévité du système.
De plus, il n'est pas possible d'avoir un amortissement satisfaisant avec un ressort à spire dans un encombrement aussi restreint; la courbe effort déplacement n'est absolument pas appropriée et ne répond pas au problème posé.
C'est pourquoi, notre invention répond à ces inconvénients majeurs grâce à un amortisseur viscoélastique approprié dans un encombrement restreint et en calibrant des jeux fonctionnels permettant d'éviter les frottements permanents entre les moyens composant notre invention.

Le brevet FR 2 884136 A1 décrit un système de stabilisation dynamique interépineux comportant des moyens rigides associé avec un amortisseur viscoélastique. Cependant, les moyens mobiles de ce dispositif ne disposent d'aucun jeu fonctionnel, particulièrement l'élément viscoélastique qui n'a pas d'espace pour se déformer risquant de se fissurer sous l'action des charges mécaniques transmises par le piston.

Notre invention répond à ces inconvénients en permettant à l'amortisseur de travailler dans des conditions optimales et en évitant des contacts permanents entre des pièces mobiles grâce aux jeux fonctionnels spécialement calibrés.

Les dessins servant à comprendre l'invention sont :

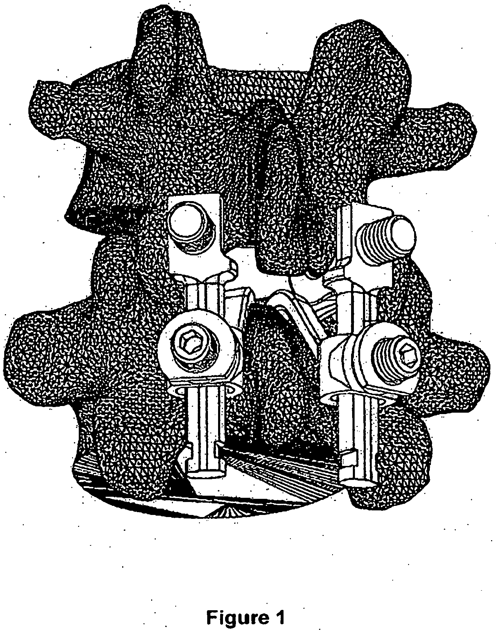

Figure 1 planche 1/8, vue isométrique d'un dispositif de l'art antérieur
Figure 2 planche 2/8, vue de profil du dispositif à implanter
Figure 3 planche 2/8, vue en perspective du dispositif à implanter
Figure 4 planche 3/8, vue en coupe de profil du dispositif à implanter
Figure 5 planche 3/8, vue éclatée montrant les différents moyens du dispositif à implanter
Figure 6 planche 4/8, vue de face du dispositif fixé sur un rachis.
Figure 7 planche 5/8, vue en coupe de profil du dispositif à implanter au repos, sans contrainte
Figure 8 planche 5/8, vue en coupe de profil du dispositif à implanter, sollicité en compression
Figure 9 planche 5/8, vue en coupe de profil du dispositif à implanter, sollicité en flexion
Figure 10 planche 5/8, vue en coupe de profil du dispositif à implanter, sollicité en extension
Figure 11 planche 6/8, vue de face du dispositif fixé sur un rachis en flexion latérale gauche
Figure 12 planche 6/8, vue de face du dispositif fixé sur un rachis en position neutre
Figure 13 planche 6/8, vue de face du dispositif fixé sur un rachis en flexion latérale droite
Figure 14 planche 7/8, vue de profil du dispositif fixé sur un rachis en flexion
Figure 15 planche 7/8, vue de profil du dispositif fixé sur un rachis en position neutre
Figure 16 planche 7/8, vue de profil du dispositif fixé sur un rachis en extension
Figure 17 planche 7/8, vue de détail, de face, du dispositif fixé sur un rachis en flexion latérale gauche
Figure 18 planche 8/8, vue de détail, de face, du dispositif fixé sur un rachis en position neutre
Figure 19 planche 8/8, vue de détail, de face, du dispositif fixé sur un rachis en flexion latérale droite
Figure 20 planche 8/8, vue de détail, de profil, du dispositif fixé sur un rachis en flexion
Figure 21 planche 8/8, vue de détail, de profil, du dispositif fixé sur un rachis en position neutre
Figure 22 planche 8/8, vue de détail, de profil, du dispositif fixé sur un rachis en extension

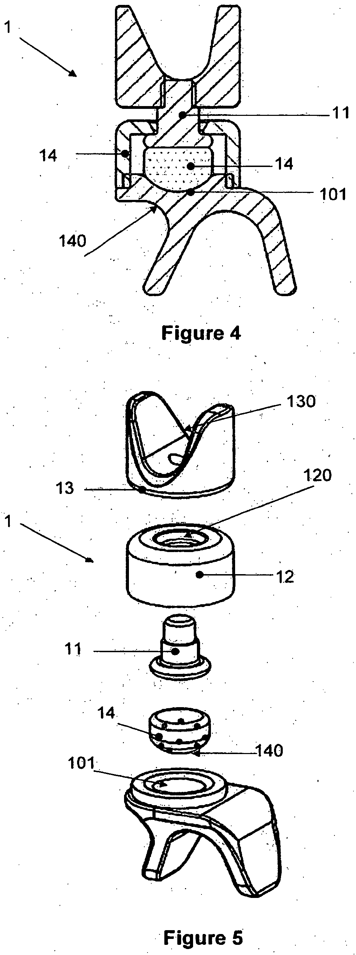

Le dispositif 1 comprend cinq moyens essentiels, dont quatre sont rigides et dont au moins un est viscoélastique (figure 4 et 5).

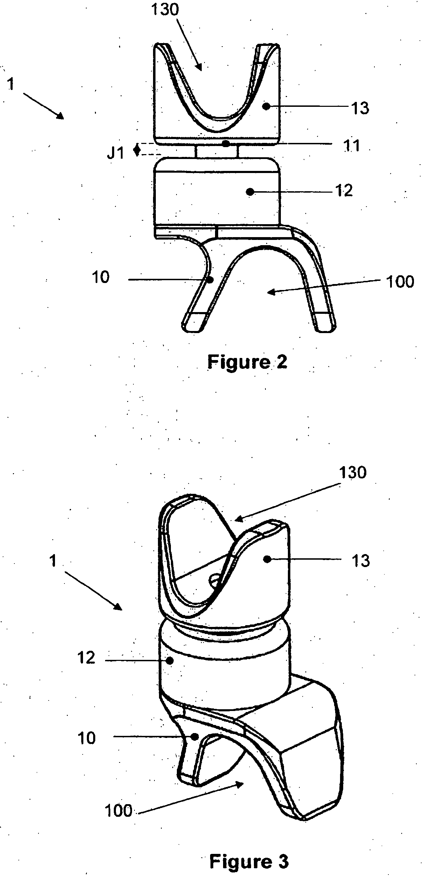

Ce sont :
- au moins un moyen viscoélastique 14 jouant le rôle d'amortisseur multiaxial avec une force de rappel élastique.
- une embase 10 comprenant un crochet inférieur 100 (figure 2 et 3) fait pour s'adapter à la géométrie de la lame de la vertèbre inférieure ; l'axe du crochet 100 de cette embase est décalé d'une valeur x comprise entre 0 et 15 mm pour obtenir une bonne fixation sur la vertèbre en respectant l'anatomie rachidienne.
- un piston rigide 11, fixé à un crochet supérieur 13, traverse un corps cylindrique creux 12 par un orifice 120 et prend appui sur le moyen viscoélastique 14 un crochet supérieur rigide 13 possédant un évidement 130 (figure 4) qui s'emboîte avec la lame de la vertèbre supérieure, ce crochet étant lié au piston rigide 11.
- un corps cylindrique creux 12 qui sert de capot protecteur au moyen viscoélastique 14 ainsi que de butée mécanique de sécurité au déplacement du crochet supérieur 13.

L'amortisseur viscoélastique 14 a une forme cylindrique. Sur sa face inférieure, il présente une partie convexe 140 qui épouse parfaitement une cuvette concave 101 appartenant à la partie supérieure de l'embase 10. Cette cuvette concave qui fait partie intégrante de l'embase 10 permet un montage parfait par auto centrage du moyen viscoélastique 14 sur ladite embase 10.

Un certain nombre de jeux J1, J2, J3 (figure 4) sont essentiels. Ils ont été déterminés pour éviter de détériorer le moyen viscoélastique 14 et obtenir une mobilité polyaxiale du crochet supérieur 13 en évitant aussi tout blocage du dispositif par compression ou déformation excessive du moyen 14.

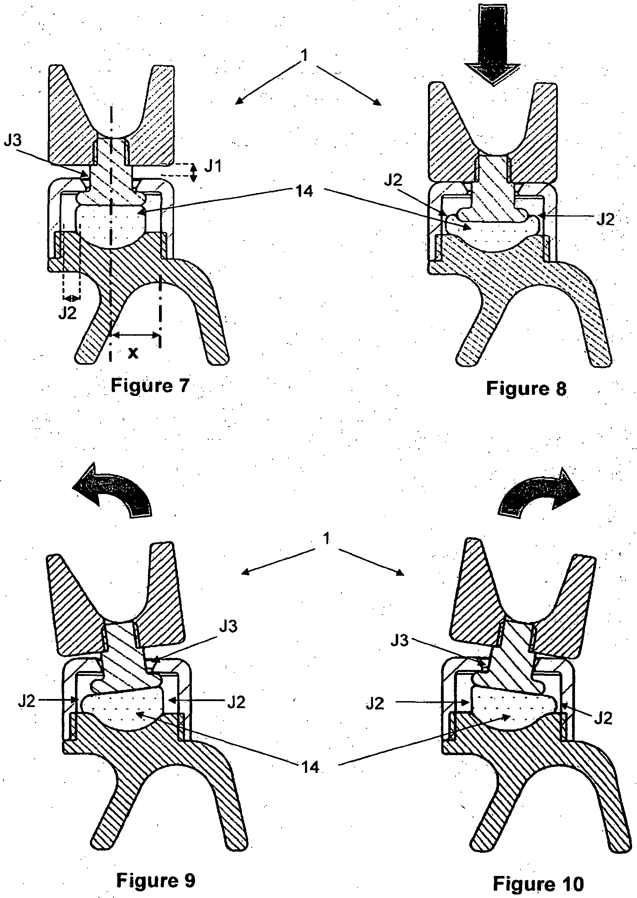

Le premier jeu J1 (figure 7) est défini par l'espace entre la face inférieure du crochet 13 et la face supérieure du corps cylindrique 12, autorise une compression verticale du moyen viscoélastique 14 lors de sollicitations mécaniques extrêmes.

Le deuxième jeu J2 (figures 7,8,9 et 10), espace résiduel défini entre les parois internes du corps cylindrique 12 et l'enveloppe du moyen viscoélastique 14 autorise une déformation sans entraîner des contraintes trop élevées du dit moyen viscoélastique 14, en évitant aussi des frottements excessifs dudit moyen 14, sur les parois internes du corps cylindrique 12. Ce jeu permet la libre déformation du moyen viscoélastique 14 à l'intérieur du corps cylindrique creux 12 quelles que soient les sollicitations qui s'appliquent au rachis.

Le troisième jeu J3 , espace résiduel en forme d' un anneau tronconique défini entre le diamètre de l'orifice 120 du corps cylindrique 12 et le diamètre de la tige piston 11, autorise la mobilité polyaxiale du crochet supérieur 13 ceci, toujours en adéquation avec les mouvements du rachis.

La parfaite stabilisation dynamique du dispositif 1 tient au fait que le moyen viscoélastique 14 est un cylindre plein dont la face inférieure 140 épouse parfaitement la cuvette concave 101 du crochet inférieur 10, son positionnement est toujours parfait. Ceci entraîne une parfaite répartition des charges qui s'appliquent au dispositif 1 qui fonctionne ainsi de manière polyaxiale, sans blocage, ni risque de rupture contrairement aux dispositifs de l'art antérieur qui ne sont en général que des assemblages, alors que le dispositif 1 est monobloc et ne présente aucune vis ou écrou.

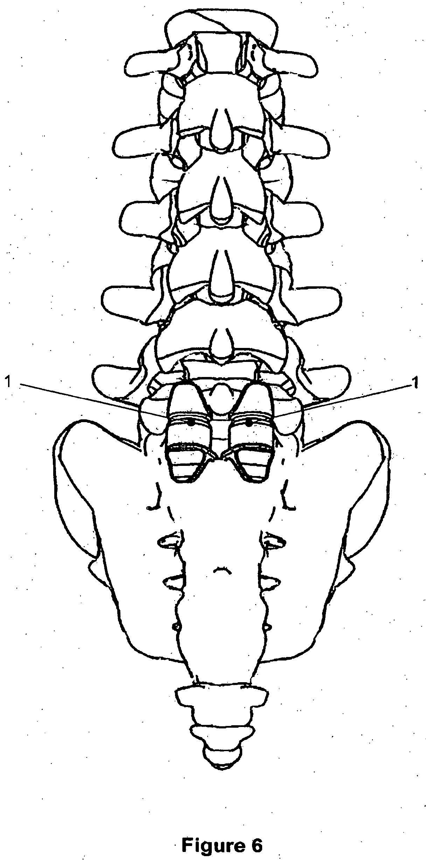

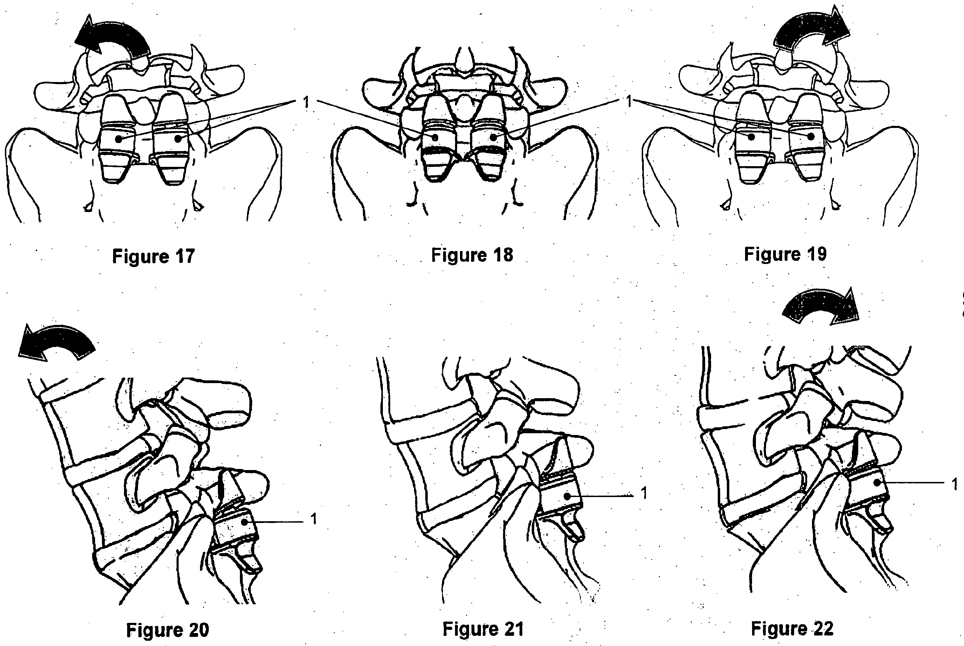

Les figures 6 à 22 montrent les divers types de mouvements subis par dispositif de stabilisation dynamique interlamaire 1.

Le premier mode de déformation (figure 8) est la compression qui déforme le moyen viscoélastique 14 sous l'action de la tige piston 11 et grâce au jeu J1. Le moyen viscoélastique 14 se déforme en compression avec la possibilité d'une expansion dans le plan horizontal en venant remplir partiellement le jeu J2, sans qu'il se produise de contact néfaste avec les parois internes du corps cylindrique 12.

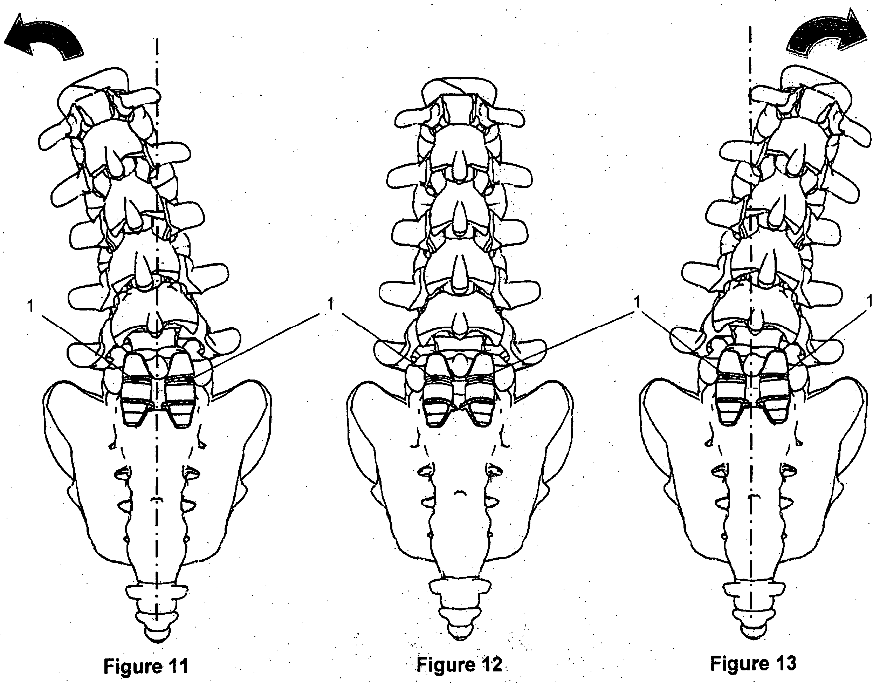

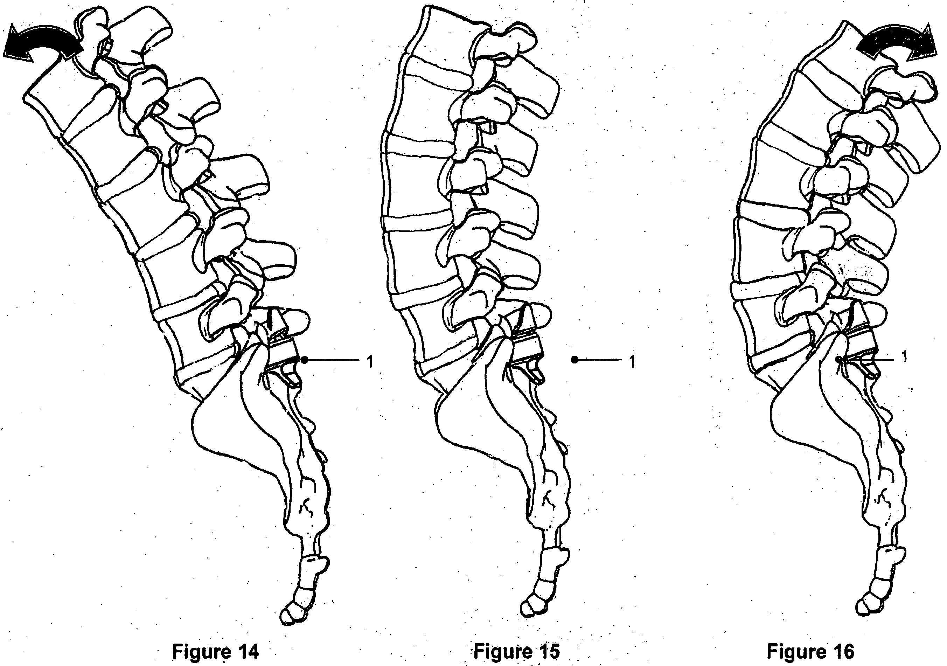

Le deuxième mode possible de déformation (figures 9 et 10) correspond à la flexion-extension (figures 14, 16, 20 et 22) et à la flexion latérale (figures 11, 13, 17 et 19) grâce à la présence combinée des jeux J1, J2 et J3. Le moyen viscoélastique 14 se déforme d'un côté sans venir au contact des parois internes du corps cylindrique 12. Il n'existe pas de décentrage du moyen viscoélastique 14 lors de ses déformations grâce à la présence de la cuvette concave 101 qui maintient centré le moyen viscoélastique 14.

Le dispositif de stabilisation dynamique interlamaire 1se fixe aux lames des vertèbres deux par deux, symétriquement, de chaque côté de l'axe vertical de la colonne vertébrale pour rétablir l'équilibre de l'articulation intervertébrale.

La présence du moyen viscoélastique 14 permet une mise en palace précontrainte en compression du dispositif de stabilisation dynamique interlamaire 1 entre les lames des vertèbres. Il agit alors comme un écarteur dynamique intervertébrale permettant de maintenir un espacement entre les vertèbres en les replaçant en position normale et en supprimant ainsi les tassements.

## Revendications

1. Dispositif de stabilisation dynamique interlamaire (1) comprenant :
- au moins un moyen viscoélastique (14) jouant le rôle d'amortisseur multiaxial avec une force de rappel élastique.
- une embase (10) comprenant un crochet inférieur 100 s'adaptant à la géométrie de la lame de la vertèbre inférieure
- un piston rigide (11) fixé à un crochet supérieur 13, traversant un corps cylindrique creux 12 par un orifice 120, et s'appuyant sur le moyen viscoélastique (14)
- un crochet supérieur rigide (13) lié au piston rigide (11), s'emboîtant avec la lame de la vertèbre supérieure ***caractérisé en ce qu'**il comprend :*
- un corps cylindrique creux (12), servant de capot protecteur au moyen viscoélastique (14) et de butée mécanique de sécurité au déplacement du crochet supérieur (13)
- ledit moyen viscoélastique (14) de forme cylindrique présentant sur sa face inférieure une partie convexe (140) s'auto centrant dans une cavité (101) intégrée dans l'embase (10),
- un jeu fonctionnel (J1), espace défini entre la face inférieure du crochet (13) et la face supérieure du corps cylindrique creux (12), particulièrement calibré au déplacement limité en compression du crochet supérieur (13),
- un jeu fonctionnel (J2) espace défini entre les parois internes du corps cylindrique creux (12) et l'enveloppe du moyen viscoélastique (14) particulièrement calibré à la déformation sans contact avec les parois internes du corps cylindrique creux.
- un jeu fonctionnel (J3), espace résiduel en forme d'un anneau tronconique défini entre le diamètre de l'orifice 120 du corps cylindrique 12 et le diamètre du piston rigide (11) particulièrement calibré au débattement nécessaire entre les crochets (100) et (13),
ces moyens une fois assemblés forment le dispositif (1) qui devient monobloc ce qui empêche tout risque de glissement, de désassemblage ou de dislocation entre les différents moyens et permet la libre déformation du moyen viscoélastique (14) à l'intérieur du corps cylindrique creux (12), tout en préservant son intégrité; il permet également que les crochets inférieurs et supérieurs (100) et (13) du dispositif (1) soient toujours fixées aux vertèbres, sécurisant les mouvements intervertébraux et les amortissements des charges mécaniques appliquées grâce à la mobilité polyaxiale du crochet supérieur (13) et à la butée de sécurité sur la face supérieure du corps cylindre creux (12)

2. Dispositif de stabilisation dynamique interlamaire (1) selon l'une quelconque des précédentes revendications, **caractérisé en ce qu'**il comprend deux dispositif monoblocs (1) qui se fixent par paire sur les vertèbres, chaque dispositif (1) étant placé de chaque côté dé l'axe vertical de la colonne vertébrale pour rétablir l'équilibre de l'articulation intervertébrale.

## Patentansprüche

1. Dynamische Vorrichtung (1) zur interlaminaren Stabilisierung, die Folgendes umfasst:
- mindestens ein viskoelastisches Mittel (14), das die Aufgabe des multiaxialen Dämpfers mit einer elastischen Rückstellkraft übernimmt,
- einen Sockel (10), der einen unteren Haken (100) umfasst, der sich an die Geometrie des Fortsatzes der unteren Wirbel anpasst,
- einen starren Kolben (11), der an einem oberen Haken (13) befestigt ist, der einen zylindrischen Hohlkörper (12) durch eine Öffnung (120) durchquert und sich auf das viskoelastische Mittel (14) stützt,
- einen oberen starren Haken (13), der mit dem starren Kolben (11) verbunden ist, indem er sich mit dem Fortsatz der oberen Wirbel verschachtelt, **dadurch *gekennzeichnet*, dass** er Folgendes umfasst:
- einen zylindrischen Hohlkörper (12), der als Schutzhaube für das viskoelastische Mittel (14) und als mechanischer Sicherheitsanschlag für die Bewegung des oberen Hakens (13) dient,
- wobei das viskoelastische Mittel (14) mit zylindrischer Form auf seiner unteren Seite einen konvexen Teil (140) aufweist, der sich selbsttätig in einem in den Sockel (10) integrierten Hohlraum (101) zentriert,
- ein funktionales Spiel (J1), Raum, der zwischen der Unterseite des Hakens (13) und der Oberseite des zylindrischen Hohlkörpers (12) definiert ist, der insbesondere für die in Kompression begrenzte Bewegung des oberen Hakens (13) kalibriert ist,
- ein funktionales Spiel (J2), Raum, der zwischen den Innenwänden des zylindrischen Hohlkörpers (12) und dem Mantel des viskoelastischen Mittels (14) definiert ist, der insbesondere für die berührungslose Verformung mit den Innenwänden des zylindrischen Hohlkörpers kalibriert ist,
- ein funktionales Spiel (J3), Restraum in Form eines kegelstumpfförmigen Rings, der zwischen dem Durchmesser der Öffnung (120) des zylindrischen Körpers (12) und dem Durchmesser des starren Kolbens (11) definiert ist, der insbesondere für das erforderliche Ausschlagen zwischen den Haken (100) und (13) kalibriert ist,
wobei diese Mittel, nachdem sie zusammengefügt sind, die Vorrichtung (1) bilden, die einstückig wird, was jede Gefahr von Gleiten, Trennen oder Ausrenken zwischen den unterschiedlichen Mitteln verhindert und die freie Verformung des viskoelastischen Mittels (14) im Inneren des zylindrischen Hohlkörpers (12) erlaubt und gleichzeitig dessen Unversehrtheit wahrt, auch ermöglicht, dass der untere und der obere Haken (100) und (13) der Vorrichtung (1) immer an den Wirbeln befestigt sind, indem sie die Zwischenwirbelbewegungen absichern und die Dämpfung der angelegten mechanischen Lasten dank der mehrachsigen Beweglichkeit des oberen Hakens (13) und des Sicherheitsanschlags auf der Oberseite des zylindrischen Hohlkörpers (12) absichern.

2. Dynamische Vorrichtung zur interlaminaren Stabilisierung (1) nach einem der vorhergehenden Ansprüche, **dadurch *gekennzeichnet*, dass** sie zwei einstückige Vorrichtungen (1) umfasst, die in Paaren auf den Wirbeln befestigt werden, wobei jede Vorrichtung (1) auf jeder Seite der vertikalen Achse der Wirbelsäule platziert ist, um das Gleichgewicht der Zwischenwirbelgelenke wiederherzustellen.

## Claims

1. Device for dynamic interlaminar stabilization (1) comprising:
- at least one viscoelastic mean (14) used as multiaxial shock absorber with an elastic return force,
- A base (10) comprising a lower hook (100) fitting to the geometry of the laminar of the lower vertebra,
- A rigid piston (11) fixed to an upper hook (13), crossing a hollow cylindrical body (12) by an opening 120, and leaning on the viscoelastic mean (14)
- A stiff upper hook (13) connected to the rigid piston (11), fitting with the laminar of the superior vertebra ***characterized* by** the following properties:
- A hollow cylindrical body (12), serving as protective cap of the viscoelastic means (14) and as a stop in the displacement of the upper hook (13)
- The said viscoelastic mean (14) with cylindrical shape presenting on its lower face a convex part (140) centering itself in a cavity (101) integrated into the base (10),
- A functional room (J1), defined between the lower face of the hook (13) and the superior face of the hollow cylindrical body (12), particularly calibrated to the limited movement in compression of the upper hook (13),
- A functional room (J2), space defined between the internal walls of the hollow cylindrical body (12) and the shape of the viscoelastic mean (14) particularly calibrated to the deformity without coming into contact with the internal walls of the hollow cylindrical body.
- A functional room (J3), residual space in the shape of a tronconic ring defined between the diameter of the opening (120) of the hollow cylindrical body (12) and the diameter of the rigid piston (11) particularly calibrated to the necessary movements between the hooks (100) and (13),
these means, after they are joined together, form the device (1) which is in one piece, what prevents any risk of sliding, dismantling or dislocation between the different means and allows the free deformation of the viscoelastic mean (14) inside the hollow cylindrical body (12), while insuring its integrity; even allowing that the lower and superior hooks (100) and (13) of the device (1) are always attached to the vertebrae, securing the intervertebral movements and the shock absorption of the applied mechanical loads thanks to the polyaxial movements of the upper hook (13) and to the security stop on the superior face of the hollow cylindrical body (12).

2. Device for dynamic interlaminar stabilization (1) according to any of the previous claims, ***characterized* in** the fact it includes two single piece devices (1) which are attached in pair on vertebrae, each device (1) on each side of the vertical axis of the spinal column is placed to restore the balance of the intervertebral joints.
